(19) Europäisches Patentamt
European Patent Office
Office européen des brevets

(11) Veröffentlichungsnummer: **0 171 584**
**B1**

# (12) EUROPÄISCHE PATENTSCHRIFT

(45) Veröffentlichungstag der Patentschrift: **12.10.88**

(51) Int. Cl.⁴: **C 07 D 233/00**

(21) Anmeldenummer: **85108322.0**

(22) Anmeldetag: **05.07.85**

(54) **Verfahren zur Herstellung von 2-Alkyl-4,5-dihydroximethylimidazolen.**

(30) Priorität: **14.07.84 DE 3426081**

(43) Veröffentlichungstag der Anmeldung:
**19.02.86 Patentblatt 86/8**

(45) Bekanntmachung des Hinweises auf die Patenterteilung:
**12.10.88 Patentblatt 88/41**

(84) Benannte Vertragsstaaten:
**BE CH DE FR GB IT LI NL**

(56) Entgegenhaltungen:
**EP-A-0 006 102**
**EP-A-0 012 371**
**DE-A-2 618 756**
**US-A-4 298 748**

**CHEMICAL ABSTRACTS, Band 86, Nr. 11, 14. März 1977, Seite 617, no. 72635j, Columbus, Ohio, US; & JP - A - 76 91 262 (SHIKOKU-KASEI KOGYO CO., LTD.) 10-08-1976**
**ULLMANN'S Encyklopädie der technischen Chemie, 4. Aufl. 1976, Verlag Cherme, Bd. 11, S. 690**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Kempe, Uwe, Dr., Daziger Strasse 9, D-6701 Dannstadt- Schauernheim (DE)**
Erfinder: **Dockner, Toni, Dr., Grossgasse 6, D-6701 Meckenheim (DE)**

**EP 0 171 584 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Diese Erfindung betrifft ein Verfahren zur Herstellung von 2-Alkyl-4,5-dihydroximethylimidazolen durch Umsetzung von 2-Alkylimidazolen mit Formaldehyd.

2-Alkyl-4,5-dihydroximethylimidazole sind gesuchte Zwischenprodukte für die Herstellung von Farbstoffen und Wirkstoffen. Man stellt sie z. B. durch Reduktion der entsprechenden Imidazolcarbonsäuren her. Monohydroximethylimidazole, wie das als Pharmavorprodukt bekannte 4-Methyl-5-hydroximethylimidazol, werden z. B. nach den Angaben der EP-PS 4 534 durch Umsetzung von 4-Methylimidazol mit Formaldehyd in alkalischem Milieu erhalten. Man hat auch schon 4,5-Dihydroximethylimidazole, die in 2-Stellung Arylgruppen enthalten, durch Behandlung der entsprechenden 2-Arylimidazole mit Formaldehyd hergestellt. Bei diesem in der DE-OS-2 618 756 beschriebenen Verfahren wird bevorzugt in wäßrigem Medium bei pH-Werten von mindestens 7 gearbeitet. Es wird darauf hingewiesen, daß man bei der Umsetzung von Imidazolen, die in 2-Stellung keine Arylgruppe enthalten, mit Formaldehyd instabile 1-Hydroximethylimidazole erhält (s. S. 6, Zeilen 8 bis 13).

Es wurde nun gefunden, daß man 2-Alkyl-4,5-dihydroximethylimidazole der Formel

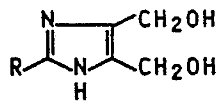

I,

in der R einen Alkylrest mit 1 bis 17 C-Atomen bedeutet, der noch Reste der Formel R'O-, $R'_2N$- oder R'S- enthalten kann, und R' für eine Alkylgruppe mit 1 bis 4 C-Atomen steht, besonders vorteilhaft dadurch herstellen kann, daß man ein Imidazol der Formel

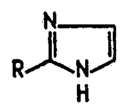

II,

in der R die oben genannte Bedeutung hat, bei Temperaturen von 40 bis 180°C mit mindestens 2 Mol Formaldehyd in Gegenwart von Basen umsetzt wobei man die Umsetzung in einer wäßrigen Lösung des Formaldehyds vornimmt.

Nach dem neuen Verfahren erhält man die 2-Alkyl-4,5-dihydroximethylimidazole in guter Ausbeute. Dieses vorteilhafte Ergebnis ist sehr überraschend, da aufgrund der Angaben in der DE-OS-2 618 756 über das Reaktionsverhalten von Imidazolen, nicht damit gerechnet werden konnte, daß sich 2-Alkyl-imidazole mit Formaldehyd unter basischen Bedingungen zu 2-Alkyl-4,5-dihydroximethylimidazolen umsetzen lassen.

Die als Ausgangsstoffe geeigneten Imidazole der Formel II enthalten in 2-Stellung einen Alkylrest mit 1 bis 17 C-Atomen, wie einen Methyl-, Ethyl-, Propyl-, Butyl- oder Heptadecylrest. Der Alkylrest kann noch durch Alkylether-, Alkylthioether- oder Dialkylaminogruppen substituiert sein, in denen die Alkylreste z. B. 1 bis 4 C-Atome enthalten. Beispielsweise seien die folgenden 2-Alkylimidazole genannt: 2-Methylimidazol, 2-Ethylimidazol und 2-Isopropylimidazol.

Die 2-Alkylimidazole werden mit mindestens 2 Mol, vorzugsweise 2 bis 3 Mol Formaldehyd, bezogen auf 1 Mol des Imidazols, umgesetzt. Der Formaldehyd wird als wäßrige Formaldehydlösung, zweckmäßigerweise in Form von technischen wäßrigen Lösungen mit Formaldehydgehalten von 10 bis 30 Gew.-%, eingesetzt. Die Gegenwart von Methanol, wie sie in technischen Formaldehydlösungen enthalten ist, stört dabei nicht. Beim erfindungsgemäßen Verfahren nimmt man die Umsetzung der Imidazole mit dem Formaldehyd in Gegenwart von Basen vor. Dabei sollte der pH-Wert des Reaktionsgemisches mindestens 7, vorzugsweise 7 bis 13 betragen. Die Reaktionstemperaturen liegen im Bereich von 40 bis 180°C, der Reaktionsdruck spielt keine Rolle.

Als Basen kommen z. B. die Hydroxide der Alkali- oder Erdalkalimetalle, wie NaOH oder KOH in Betracht. Man kann als Basen auch tertiäre Amine, wie Triethylamin, Tri-n-butylamin oder Methyldibutylamin verwenden. Die als Katalysator wirkende Base wird z. B. in Mengen von 0,1 bis 1,0, bevorzugt 0,3 bis 0,7 Mol pro Mol Imidazol eingesetzt. Bei Verwendung der Alkali- oder Erdalkalihydroxide hält man den anfangs eingestellten pH-Wert durch Zugabe von weiteren Anteilen der Base konstant.

Nach der Umsetzung, die nach etwa 20 bis 60 Stunden abgeschlossen ist, isoliert man die Dimethylolverbindungen aus dem Reaktionsgemisch z. B. nach Abdestillieren der flüchtigen Bestandteile. Technisch besonders vorteilhaft ist hierbei, daß die flüchtigen tertiären Amine wieder erneut eingesetzt werden können.

Die nach dem Verfahren der Erfindung erhältlichen 2-Alkyl-4,5-dihydroximethylimidazole sind wertvolle Zwischenprodukte für Farb- und Kunststoffe sowie für Spezialchemikalien. Die erhaltenen Verbindungen wurden durch magnetische Kernresonanz und Elementaranalyse, wie angegeben, identifiziert.

**Beispiel 1**

2-Methyl-4,5-dihydroximethylimidazol

82 g (1 Mol, 2-Methylimidazol und 220 g (2,2 Mol) 30 gewichtsprozentige wäßrige Formaldehydlösung werden 1 Stunde bei 60°C gerührt. Man gibt zu dem Gemisch 50,4 g (0,49 Mol) Triethylamin und rührt 48 Stunden unter Rückfluß (Temperatur ca. 78°C). Die Lösung wird im Vakuum bei einer Badtemperatur von 60°C bis zur Trockne eingeengt. Dabei werden 160 g Rohprodukt erhalten. Das Rohprodukt wird mit 128 g Methanol und 32 g Isopropanol je 30 Minuten bei 50°C und bei 10°C unter Kühlen gerührt. Dann wird der farblose Feststoff abfiltriert, zweimal mit je 200 ml Aceton gewaschen und 12 Minuten bei 45°C getrocknet. Man erhält 91,6 g 2-Methyl-4,5-dihydroximethylimidazol vom Schmelzpunkt 182 bis 182,5°C. Das entspricht einer Ausbeute von 64,5 %. Die Reinheit (HPLC) beträgt 99,8 %.

| Elementaranalyse: | | C | H | N | O |
|---|---|---|---|---|---|
| | Ber. | 50,7 | 7,1 | 19,7 | 22,5 % |
| | Gef. | 50,6 | 7,6 | 19,7 | 22,1 % |

Massenspektrum: M+ 142

$^1$H-NMR-Spektrum (Lösungsmittel DDMSO; TMS int. Standard, Angabe in ppm): 2,18 (s, 3p) 4,35 (s, 4p).

**Beispiel 2**

2-Ethyl-4,5-dihydroximethylimidazol

Man verfährt wie im Beispiel 1 angegeben, wobei man jedoch anstelle von 2-Methylimidazol 96 g (1 Mol) 2-Ethylimidazol verwendet. Dabei werden 155 g Rohprodukt erhalten. 155 g Rohprodukt werden mit 52 g Methanol 30 Minuten bei 50°C und 10°C unter Kühlen gerührt. Nach dem Filtrieren, Nachwaschen mit zweimal 150 ml Aceton und Trocknen im Trockenschrank erhält man 117,5 g 2-Ethyl-4,5-dihydroximethylimidazol vom Schmp. 173,35 bis 174,0°C. Dies entspricht einer Ausbeute von 75,4 %. Die Reinheit dieser Probe nach HPLC beträgt 99,8 %.

| Elementaranalyse: | | C | H | N | O |
|---|---|---|---|---|---|
| | Ber. | 53,9 | 7,7 | 17,9 | 20,5 % |
| | Gef. | 54,0 | 7,8 | 17,7 | 20,5 % |

Massenspektrum: M+ 156

$^1$H-NMR-Spektrum (Lösungsmittel DDMSO) 1,15 (t, ep) 2,55 (q, 2p) 4,4 (s, 4p).

**Beispiel 3**

2-Isopropyl-4,5-dihydroximethylimidazol

110 g (1 Mol) 2-Isopropylimidazol und 220 g (2,2 Mol) einer 30 %-igen wäßrigen Formaldehydlösung werden 1 Stunde bei 60°C gerührt. Man gibt zu dem Gemisch 50,4 g (0,49 Mol) Triethylamin und rührt unter Rückfluß bei ca. 78°C. Das Produkt kristallisiert aus dem Ansatz aus. Nach 48 Stunden wird der Ansatz auf 5°C abgekühlt. Das Produkt wird nach 1 Stunde durch Filtration isoliert. Man erhält 136,8 g Rohprodukt, das zweimal mit je 200 ml Aceton gewaschen wird. Nach dem Trocknen im Vakuumtrockenschrank bei 45°C erhält man 126,1 g 2-Isopropyl-4,5-dihydroximethylimidazol vom Schmp. 199,5 bis 201,0°C. Durch Einengen des Filtrats erhält man 107 g eines Rohprodukts, aus dem durch Anrühren mit 107 g Methanol bei 50°C über 30 Minuten und anschließendes Kühlen bei 5°C über 30 Minuten nach Filtrieren und Trocknen weitere 18,5 g 2-Isopropyl-4,5-dihydroximethylimidazol erhalten werden können. Die Gesamtausbeute beträgt 84,8 %.

| Elementaranalyse: | | C | H | N | O |
|---|---|---|---|---|---|
| | Ber. | 56,5 | 8,2 | 16,5 | 18,8 % |
| | Gef. | 56,7 | 8,6 | 16,5 | 18,2 % |

Massenspektrum: M+ 170

$^1$H-NMR (Lösungsmittel DDMSO):

1,12 1,24 (d, 6p) 2,9 (Septett, 1p) 4,35 (s, 4p)

**Beispiel 4**

2-Ethyl-4, 5-dihydroximethylimidazol

Man verfährt wie im Beispiel 2 beschrieben, wobei man jedoch 90,7 g (0,49 Mol, Tri-n-butylamin anstelle von Triethylamin einsetzt. Nach 46 Stunden werden 10 g Formaldehyd (als 30 %-ige wäßrige Lösung) zugesetzt. Nach insgesamt 62 Stunden bei 110°C wird die obere Phase abgetrennt und das Produkt durch Einengen isoliert. Man erhält nach der in Beispiel 2 beschriebenen Aufarbeitung 139,2 g 2-Ethyl-4,5-dihydroximethylimidazol vom Schmp. 173 bis 174°C (Ausbeute 89,1 %).

**Beispiel 5**

2-Methyl-4,5-dihydroximethylimidazol

41 g 2-Methylimidazol werden in 20 ml Wasser gelöst. Zu der Lösung gibt man langsam bei 15 bis 25°C 100 g einer 30%-igen wäßrigen Formaldehydlösung. Mit 9 ml einer 40 %-igen wäßrigen Kaliumhydroxidlösung wird ein pH von 12,4 eingestellt. Durch Zugabe einer Lösung aus Kaliumhydroxid und wäßrigem Formaldehyd (Molverh. 1 : 2) wird während der Reaktion der pH bei 12,4 gehalten. Nach 28 Stunden wird der Ansatz eingeengt und das Rohprodukt, wie bei Beispiel 1 beschrieben, behandelt. Man erhält 55,7 g 2-Methyl-4,5-dihydroximethylimidazol (Ausbeute 78,4 %) vom Schmp. 182 bis 182,5°C.

Man verfährt wie im ersten Absatz beschrieben, wobei man jedoch anstelle der Kalilauge 8 ml einer 40 %-igen Natronlauge zugibt, womit man einen pH-Wert von 12,1 einstellt. Nach 28 Stunden wird der Ansatz eingeengt und wie in Beispiel 1 angegeben aufgearbeitet. Man erhält 50 g 2-Methyl-4,5-dihydroximethylimidazol (Ausbeute 70,2 %) vom Schmp. 182 bis 182,5°C.

**Patentansprüche**

1. Verfahren zur Herstellung von 2-Alkyl-4,5-dihydroximethylimidazolen der Formel

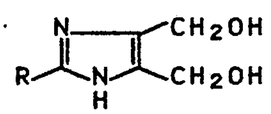

I,

in der R einen Alkylrest mit 1 bis 17 C-Atomen bedeutet, der noch Reste der Formel R'O-, $R'_2N$ oder R'S- enthalten kann, und R' für eine Alkylgruppe mit 1 bis 4 C-Atomen steht, dadurch gekennzeichnet, daß man ein Imidazol der Formel

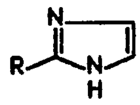

II,

in der R die obengenannte Bedeutung hat, bei Temperaturen von 40 bis 180°C mit mindestens 2 Mol Formaldehyd in Gegenwart von Basen umsetzt, wobei man die Umsetzung in einer wäßrigen Losung des Formaldehyds vornimmt.

2. Verfahren nach Anspruch 1, dadurch gekennzeichnet, daß man die Base in einer Menge von 0,1 bis 1 Mol je Mol Imidazol anwendet.

3. Verfahren nach Anspruch 1, dadurch gekennzeichnet, daß man als Base ein Hydroxid der Alkali- oder Erdalkalimetalle verwendet.

4. Verfahren nach Anspruch 1, dadurch gekennzeichnet, daß man als Base ein tertiäres Amin verwendet.

**Claims**

1. A process for the preparation of a 2-alkyl-4,5-dihydroxymethylimidazole of the formula

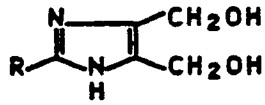

I,

where R is alkyl of 1 to 17 carbon atoms which may furthermore contain radicals of the formula R'O-, $R'_2N$- or R'S-, and R' is alkyl of 1 to 4 carbon atoms, wherein an imidazole of the formula

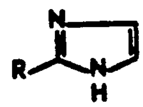

II,

where R has the above meanings, is reacted with not less than 2 moles of formaldehyde at from 40 to 180°C in the presence of a base, the reaction being carried out in an aqueous solution of the formaldehyde.

2. A process as claimed in claim 1, wherein the base is used in an amount of from 0.1 to 1 mole per mole of imidazole.

3. A process as claimed in claim 1, wherein the base used is a hydroxide of an alkali metal or an alkaline earth metal.

4. A process as claimed in claim 1, wherein the base used is a tertiary amine.

**Revendications**

1. Procédé de préparation de 2-alkyl-4,5-dihydroxyméthylimidazoles de formule

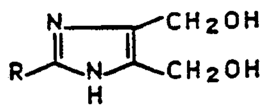

I,

dans laquelle R est un radical alkyle contenant 1 à 17 atomes de carbone et pouvant encore contenir des restes de formules R'O-, $R'_2N$- ou R'S-, le radical R' étant un groupe alkyle contenant 1 à 4 atomes de carbone, caractérisé en ce que l'on fait réagir un imidazole de formule

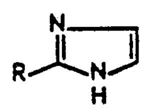

II,

dans laquelle R a la signification sus-indiquée, à des températures de 40 a 180°C, avec au moins 2 moles de formaldéhyde en présence de bases, la réaction étant effectuée dans une solution aqueuse du formaldéhyde.

2. Procédé suivant la revendication 1, caractérisé en ce que l'on utilise la base en une quantité de 0,1 à 1 mole par mole d'imidazole.

3. Procédé suivant la revendication 1, caractérisé en ce que l'on utilise comme base un hydroxyde des métaux alcalins ou alcalino-terreux.

4. Procédé suivant la revendication 1, caractérisé en ce que l'on utilise comme base une amine tertiaire.